# EUROPEAN PATENT SPECIFICATION

(11) **EP 0 567 532 B1**
(45) Date of publication and mention of the grant of the patent: **24.07.1996**
(21) Application number: 92903564.0
(22) Date of filing: 16.01.1992
(51) Int. Cl.: C12N 5/10, C12P 21/08, C12Q 1/00, C12N 15/00, G01N 33/00, C07K 16/18

(54) **A METHOD OF PRODUCING AUTOANTIBODIES**
METHODE ZUR PRODUKTION VON AUTOANTIKÖRPERN
PROCEDE DE PRODUCTION D'AUTO-ANTICORPS

(30) Priority: 18.01.1991 DK 90/91
(43) Date of publication of application: 03.11.1993
(73) Proprietor: NOVO NORDISK A/S, 2880 Bagsvaerd (DK)
(72) Inventor: DYRBERG, Thomas, Peter, DK-2900 Hellerup (DK); PETERSEN, Jacob, Steen, DK-2300 Copenhagen S (DK)
(86) International application number: DK9200012
(87) International publication number: WO9213065

(56) References cited:
- EP-A- 0 322 240
- WO-A-89/12823
- WO-A-91/16910
- NATURE, vol. 348, December 1990; H. KANESHIMA et al., pp. 561-562
- SCIENCE, vol. 241, 1988; J.M. McCUNE et al., pp. 1632-1639
- DIALOG INFORMATION SERVICES, File 154, Medline 66-90 May, Medline AN 90031730; D.E. MOSIER et al., AN 07124730
- NATURE, vol. 335, September 1988; D.E. MOSIER et al., pp. 256-259
- DIALOG INFORMATION SERVICES, File 154, Medline 66-90/May, Medline AN 91006288; H. TIGHE et al., AN 07487288
- DIALOG INFORMATION SERVICES, File 55: BIOSIS 68-92/May, Biosis AN 7516994; DICKEY et al.
- DIALOG INFORMATION SERVICES, File 55: BIOSIS, Biosis AN 7827480; T.D. GEPPERT et al.
- DIALOG INFORMATION SERVICES, File 55: BIOSIS, Biosis AN 7529511; W.D. DICKEY et al.
- DIALOG INFORMATION SERVICES, File 55, BIOSIS, Biosis AN 7516172; DAVIES et al.
- DIALOG INFORMATION SERVICES, File 434, SCISEARCH; M. ROWE et al., AN 10574497
- DIALOG INFORMATION SERVICES, File 55, BIOSIS, Biosis AN 40093568; J. PETERSEN et al.
- NATL. LIBRARY OF MEDICINE DATABASE, Medline, File Med 89; S.M. KRAMS et al., AN 90063459
- NATL. LIBRARY OF MEDICINE DATABASE, Medline, File Med 89; M.A. DUCHOSAL et al., AN 90354803

## Description

### FIELD OF INVENTION

The present invention relates to a method of producing autoantibodies which are reactive with an antigen characteristic of an autoimmune disease, as well as to a method of screening for substances with a therapeutic effect on autoimmune diseases.

### BACKGROUND OF THE INVENTION

For the purpose of studying the human immune system, various animal, in particular mouse, models have been constructed in which immunodeficient mice have been transplanted with human cells or tissue which form part of the immune system. A favoured mouse strain for this purpose is the severe combined immunodeficient (SCID) mouse which is lacking in T and B lymphocytes. Such mice are consequently deficient in immune functions mediated by T and B cells (for a more detailed description of the SCID mouse strain see G.C. Bosma et al., Nature 301, 10 February 1983, pp. 527-530).

Thus, J.M. McCune et al., Science 241, 23 September 1988, pp. 1632-1639 (see also EP 322 240), describe the transplantation into SCID mice of human fetal liver tissue (as a source of stem cells) as well as thymus and lymph node tissue which develops into a thymus and lymph node, respectively, so that differentiation of the stem cells into, eventually, human T and B cells takes place. It is suggested that the hybrid SCID mice may be used to produce human immunoglobulin.

D.E. Mosier et al., Nature 335, 15 September 1988, pp. 256-259, report the transfer of a functional human immune system to SCID mice by transplanting human peripheral blood leukocytes (PBL) from normal/healthy donors into SCID mice. The reconstituted mice secrete human immunoglobulin, and a specific human antibody response is elicited when the mice are immunized with tetanus toxoid. It is suggested that the reconstituted mice may be used to study the human immune response to vaccines, to test immunomodulators and to study pathogenic mechanisms in infection and neoplasia.

M.A. Duchosal et al., J. Exp. Med. 172, September 1990, pp. 985-988, describe the transfer of PBL from systemic lupus erythematosus (SLE) patients to SCID mice. The reconstituted mice produce human immunoglobulin, including antinuclear antibodies in titers which are comparable to those of the SLE donors. It is suggested to use this mouse model to study the development of SLE and to compare different potential therapies.

H. Tighe et al., Eur.J.Immunol. 20(8), 1990, pp. 1843-1848, describes the reconstitution of SCID mice with synovial or blood lymphocytes from a rheumatoid arthritis patient. Mononuclear cells from synovial fluid or tissue produce IgM rheumatoid factor. An attempt to stimulate production of rheumatoid factor autoantibodies with immune complexes of tetanus toxoid and IgG is reported to have failed.

### SUMMARY OF THE INVENTION

The present invention relates to a method of producing autoantibodies which are reactive with an antigen characteristic of an autoimmune disease, the method comprising
(a) administering lymphocytes from a donor individual suffering from an autoimmune disease to immunodeficient mammals,
(b) stimulating, after a period of time sufficient to permit reconstitution of immune cells in at least one of said mammals, autoreactive cells of said reconstituted immunodeficient mammal by subsequent immunization with one or more relevant target antigens,
(c) selecting a reconstituted mammal producing the desired autoantibodies,
(d) immortalizing B-lymphocytes of the selected mammal before or after isolating lymphocytes therefrom, and
(e) culturing the immortalized B-lymphocytes under conditions permitting their production of the desired autoantibodies and recovering the resulting autoantibodies from the culture.

In the present context, the term "autoantibodies" is understood to indicate antibodies which are the same as or functionally equivalent to antibodies whose presence in blood or tissue of an individual is indicative of a developing or established autoimmune disease, " functionally equivalent" being taken to mean that the autoantibodies are reactive with the same antigen(s) as authentic autoantibodies from individuals with a developing or established autoimmune disease. The term is intended to include antibody fragments, e.g. Fab', F(ab')₂, Fv, single-domain antibodies, or the like.

The term "immunodeficient mammal" is intended to indicate a mammal with a severely decreased immune capacity, in particular a mammal which lacks functional B and/or T cells. One common form of such a deficiency is usually termed severe combined immunodeficiency (SCID).

The term "reconstitution" is used to indicate the establishment of an at least partly functional immune system in a mammalian host by administering immune cells thereto and allowing the cells to proliferate in the host. A "reconstituted mammal" is a mammal which has been so treated.

In the course of research leading to the present invention, it has been found possible to stimulate the production of the desired autoantibodies by autoreactive cells in the reconstituted mammal by subsequent immunization (i.e. after reconstitution) of the mammal with one or more relevant target antigens in the form of isolated antigens or functional equivalents thereof or in the form of target organs, tissues or cells containing the antigens. In this connection, the term "target antigens" is intended to indicate antigens with which the autoantibodies or the cells producing the antibodies are reactive. In case the lymphocytes administered to the mammal are derived from an IDDM patient, the reconstituted mammal may suitably be stimulated with islets of Langerhans, islet β-cells or antigens derived therefrom. It should be noted that the target antigens need not be derived from the same species as the lymphocyte donor. Thus, to stimulate the production of, for instance, human autoantibodies in the reconstituted mammal, involving the administration of human lymphocytes, the mammal may be immunized with, for instance rat islets or islet cells.

The method according to the invention provides a convenient way of obtaining human autoantibodies reproducibly and in large quantities. By selecting a mammal producing the desired autoantibodies and isolating the autoantibodies from immortalized B-lymphocytes thereof, it is possible to produce autoantibodies of interest without having isolated or characterized the antigen which, in the donor individual, has given rise to the formation of the autoantibodies.

Since the autoantibodies of interest may be produced in substantially pure/isolated form, it may be possible greatly to simplify the often quite cumbersome assay procedures by which an autoimmune disease is diagnosed. In case of diabetes, the currently used procedure for diagnosing the disease in the clinically asymptomatic, prediabetic stage is to detect islet cell antibodies by indirect immunofluorescence, whereby patient serum is incubated with a section of human pancreas, followed by incubation with anti-human immunoglobulin conjugated to fluorescein isothiocyanate and detection under an ultraviolet microscope (cf., for instance, G.F. Botazzo et al., Lancet ii, 1974, pp. 1279-1282). Alternatively, an immunohistochemical procedure for detecting islet cell antibodies has been used (cf., for instance, A. Takahashi et al., Diabetologica 29, 1986, pp. 378-382; J. Krell and B.S. Rabin, Diabetes 34, 1984, pp. 300-305). In either case, the procedures employed are time-consuming and require specialized equipment to conduct.

Contrary to this, the present invention makes it possible to carry out assays which are simpler and more reliable than those currently used to diagnose autoimmune conditions. For instance, it would be possible to set up a competitive assay in which a predetermined amount of labelled autoantibody produced by the present method is incubated with a preparation containing a target antigen (i.e. an antigen which is reactive with the autoantibody) together with patient serum. A decreased amount of labelled antibody bound to the target antigen compared to a control to which no serum has been added will then indicate the presence of autoantibodies in the serum. Such an assay procedure permits a more accurate determination of the presence of autoantibodies in sera than does the use of positive patient serum as a reference standard. It also permits the amount of autoantibodies in the patient serum to be quantified. Furthermore, the autoantibodies produced by the present method are human antibodies and as such reactive with the target antigen in a fashion which is more directly comparable to the reactivity of autoantibodies in patient serum than that of, for instance, mouse antibodies.

In another aspect, the invention relates to a method of screening for substances with a therapeutic effect on an autoimmune disease, the method comprising administering a substance suspected of having a therapeutic effect on a specific autoimmune disease to an immunodeficient mammal which has been reconstituted with functional lymphocytes of a donor individual suffering from the autoimmune disease in question and to which T-cells recognising a target cell, tissue or organ have been administered, and determining any effect of the administered substance on the reactivity of said T-cells with said target cell, tissue or organ.

In the present context, the term "target cell, tissue or organ" is intended to mean a cell, tissue or organ the destruction or functional deactivation of which by T-cells gives rise to the symptoms characteristic of the autoimmune disease in question, e.g. islet β-cells in case of insulin-dependent diabetes mellitus.

T-cells (either T-cell lines or T-cell clones) recognising target cells, tissues or organs may be derived from patients suffering from an autoimmune disease (cf., for instance, B. Roep et al., Nature 345, 1990, pp. 632-634).

### DETAILED DISCLOSURE OF THE INVENTION

The method of the present invention is particularly important in that it permits the production of human autoantibodies in another mammal. Thus, in a preferred embodiment of the present method, the donor individual is a human donor. The lymphocytes administered to the immunodeficient mammal are therefore preferably obtained from human peripheral blood.

In principle, there is no limitation as to which autoimmune diseases may be diagnosed by means of the autoantibodies produced by the present method. Thus, the autoantibodies may be reactive with antigens which are characteristic of both organ-specific and systemic autoimmune diseases. Suitable examples of such autoimmune diseases are insulin-dependent diabetes mellitus, systemic lupus erythematosus, multiple sclerosis, thyroiditis, myasthenia gravis and rheumatoid arthritis. In particular, the autoimmune disease is insulin-dependent diabetes mellitus (IDDM).

As indicated above, immunodeficient mice transplanted with human tissue or cells provide a convenient animal model for the study of the human immune system. The immunodeficient mammal used in the present method is therefore preferably a mouse, in particular a SCID mouse.

Of particular interest to the present purpose, it has been found that the desired autoantibodies may be produced in a comparable, or preferably higher, concentration in the reconstituted mammal than in the serum of the lymphocyte donor individual. This indicates that the immune cells of the reconstituted mammal preferentially produce the autoantibodies of interest, permitting a higher number of autoantibody-producing B-lymphocytes to be subsequently isolated from the mammal. The concentration/titre of desired autoantibodies produced by the reconstituted mammal is suitably a least 2 times higher, more preferably at least 5 times higher, most preferably at least 10 times higher, such as about 20 times higher, than is found in the lymphocyte donor serum (as measured by, for instance ELISA or radioimmunoassay techniques).

The immortilization of the B-lymphocytes in step (d) of the method of the invention may be carried out in any conventional way of immortalizing such cells. For instance, immortalization may be carried out by inoculating the reconstituted mammal with Epstein-Barr virus (e.g. by injecting a culture medium containing the infectious virus into the mammal) before isolating B-lymphocytes from the mammal. Alternatively, immortalization may be carried out by Epstein-Barr virus transformation of B-lymphocytes isolated from the selected reconstituted mammal, e.g. according to the method described by D. Goosens et al., J. Immunol. Methods 101, 1987, pp. 193-200). Furthermore, the B-lymphocytes may be immortalized by fusion with a suitable immortal cell line after isolation from the reconstituted mammal, e.g. as described by Carroll et al., J. Immunol. Methods 89, 1986, pp. 61ff.).

Culturing the immortalized B-lymphocytes and recovery thereof from the culture (step (d) of the method of the invention) may be carried out according to conventional procedures, e.g. as described in Carroll et al, op. cit.

In the method of the invention of screening for substances with a therapeutic effect on an autoimmune disease, the functional donor lymphocytes are preferably B-lymphocytes which produce autoantibodies against the autoimmune disease in question. To ensure an increased reproducibility of the screening method, the B-lymphocytes may advantageously be immortalized B-lymphocytes, in particular lymphocytes prepared by the method of the invention as described above (steps (a)-(d)). The lymphocytes are preferably human lymphocytes. If the method of the invention is intended for screening substances useful in the treatment of diabetes, the lymphocytes should be ones derived from a diabetic patient.

To establish a test system comparable to the human immune system, the T-cells used in the screening method of the invention are preferably human cells. For a closer approximation to a complete immune system, e.g. the human immune system, it may be advantageous additionally to transfer a target cell, tissue or organ, in particular a human target cell, tissue or organ, to the immunodeficient mammal.

To provide a convenient way of determining the effect of the substance administered to the mammal on the organ or organs affected by the autoimmune disease, cells or tissue from such organ or organs may additionally be transplanted into the reconstituted mammal. To investigate the effect of the substance on a human system, human cells or tissue derived from such organs may be thus transplanted. When the autoimmune disease in question is IDDM, such cells or tissue may conveniently be islet cells or pancreatic tissue comprising islets of Langerhans.

The invention is further illustrated in the following examples which are not in any way intended to limit the scope of the invention as claimed.

### EXAMPLE 1

### Production of human immunoglobulin in SCID mice

11 male C.B-17 SCID mice, 5-6 weeks old (cf. G.C. Bosma et al., op. cit.) were injected intraperitoneally with 20x10⁶ peripheral blood mononuclear cells (PBMNC) obtained from type 1 diabetic patients within 4 months of the clinical onset of diabetes.

Human immunoglobulin (IgG and IgM) levels in the serum of the reconstituted SCID mice were determined by an antibody capture ELISA assay using microtiter plates coated with 5 µg/ml goat anti-human IgG or IgM (obtained from Zymed). Serial dilutions of the serum samples were incubated at room temperature for 2 hours. Bound Ig was quantified by means of peroxidase-conjugated goat anti-human F(ab₂) IgG, H+L (obtained from Zymed), and the immunoglobulin concentration of the samples was calculated. All the reconstituted SCID mice were shown to produce human immunoglobulins, but in highly varying concentrations (IgG: 1-2816 µg/ml; IgM: 0-344 µg/ml), reaching peak levels 8-11 weeks after the injection of PBMNC.

To stimulate autoreactive B-cells, mice selected on the basis of their human immunoglobulin levels were immunized 60 days after reconstitution with PBMNC with rat islets in Freund's complete adjuvant.

### Immunofluorescence analysis of mouse sera

Sera from these mice as well as from non-immunized mice were then tested by immunofluorescence analysis on frozen sections of human pancreas (4 µm thick, blood group O, acetone fixed) which were incubated with undiluted mouse sera for 90 minutes. After washing in PBS, the pancreas sections were incubated for 40 minutes with FITC-conjugated anti-human IgG (Dako, Denmark) diluted 1:20 in PBS, washed and mounted and subsequently viewed under a fluorescence microscope. None of the sera from reconstituted, non-immunized mice stained islets in the human pancreas sections regardless of the human IgG concentration. On the other hand, serum samples obtained from a SCID mouse on day 19 after immunization with rat islets contained islet cell cytoplasmic antibodies (ICA). The SCID mouse serum stained human pancreatic islets with an intensity which was comparable to that induced by the donor patient serum diluted 1:64.

### Immunoprecipitation analysis of mouse sera

The SCID mouse sera were also tested by immunoprecipitation analysis of rat islets to detect the presence of human autoantibodies reactive with islet cells.

Sera from recent onset type 1 diabetic patients and sera from PBMNC reconstituted SCID mice before and after immunization with neonatal rat islets as described above were used to immunoprecipitate the Triton X₁₁₄ detergent phase cytosolic fraction of [³⁵S]methionine-labelled rat islet (prepared as described in S. Bækkeskov et al., Nature 347, 13 September 1990, pp. 151-156. Immunoprecipitates were subjected to SDS-PAGE followed by fluorographic analysis as described in S. Bækkeskov et al., op. cit.

Serum from one of the patients strongly bound the 64 kD autoantigen. Serum from one of the SCID mice reconstituted with PBMNC from this patient recognized the 64 kD autoantigen, albeit weakly. Immunization with rat islets did not induce any stronger binding. On the other hand, the ICA positive SCID serum (obtained above) bound the 64 kD autoantigen with approximately the same intensity as serum from the patient even though the human IgG concentration in the mouse serum was only 512 µg/ml compared to 7823 µg/ml in the patient serum. Serum from this particular mouse obtained before immunization with rat islets (huIgG 221 µg/ml) did not precipitate the 64 kD autoantigen. These results suggest that autoreactive B-cells had survived for at least 2 months and could be stimulated by immunization of the mice with an appropriate antigen.

### Immunoblotting analysis of mouse sera

Finally, the SCID mice sera were tested by immunoblotting analysis of total lysates of neonatal rat islets. The proteins were denatured with sodium dodecyl sulphate (SDS) under reducing conditions using DTT essentially as described by Atar et al. J. Immunol. 143, 1989, p. 533, subjected to electrophoresis on a 10% polyacrylamide gel and electroblotted onto nitrocellulose filters. For immunostaining, nitrocellulose strips were incubated for 1.5 hour with sera from recent onset type 1 diabetic patients (1:50 dilution) or sera from reconstituted SCID mice (1:5 dilution) before or after immunization with neonatal rat islets, after which they were washed, incubated for 1 hour with goat anti-human F(ab₂) IgG, H+L conjugated to alkaline phosphatase (obtained from Zymed), washed and finally incubated with substrate (cf. Atar et al., op. cit.).

Several bands were recognized by the patient sera in the immunoblotting analysis. A molecule with an apparent molecular weight of 52 kD was recognized by the ICA and 64 kD autoantigen positive patient serum, but not by an ICA negative patient serum. Serum from the ICA and 64 kD autoantigen positive SCID mouse obtained after immunization with rat islets also bound the 52 kD antigen in immunoblotting, whereas a serum sample obtained the day before immunization showed no reactivity with this antigen. To further characterize this antibody reaction, the postimmune serum was adsorbed by incubating it for 60 minutes at room temperature with lysates of either rat islets (obtained by homogenising the cells in a hypotonic buffer in a Potter-Elverhjelm homogeniser, and separating the serum by centrifugation) or EBV-transformed lymphocytes before immunoblotting analysis. Pre-incubation with rat islets, but not with lymphocytes reduced the binding to the 52 kD antigen, indicating that the 52 kD molecule is not a common cellular determinant.

None of the reconstituted SCID mice, whether rat islet immunized or not, exhibited elevated blood sugar levels or impaired glucose tolerance, and examination of the pancrease from the immunized SCID mice revealed no morphological changes 40 days after immunization. These results might be explained by the brief presence of autoantibodies (less than 40 days) in the mice, or by the failure of mouse complement to fix human IgG. Alternatively, the results suggest that the islet cell autoantibodies are not in themselves sufficient to induce β-cell pathology, but may represent secondary phenomena to an autoimmune attack on the β-cells.

The experiments described above demonstrate that it is possible to stimulate the selective proliferation of autoreactive B-cell clones transferred to SCID mice.

### EXAMPLE 2

### Production of human monoclonal autoantibodies from reconstituted SCID mice

Splenocytes and thymocytes from the islet cell autoantibody positive mouse described in Example 1 were isolated by carefully pulling the tissues apart and isolating the total cell population by centrifugation. The peritoneal cells from this mouse were isolated by washing the peritoneal cavity carefully with culture medium by means of a syringe and needle and separating the cells by centrifugation. The isolated cells were then transformed with Epstein-Barr virus by incubating them in amedium containing infectious Epstein-Barr virus (cf. D. Goosens et al., op. cit.). An IgG producing cell line thus established from the splenocytes was confirmed by flowcytometry (using monoclonal antibody markers specific for human B-lymphocytes) to consist of human B-cells. Purified IgG secreted by these cells are currently being tested for islet cell reactivity. It is expected that B-cell lines established from the islet cell autoantibody positive mouse will, on closer analysis, prove to produce the islet cell antibodies.

These results suggest that SCID mice reconstituted with human PBL may prove a useful alternative source of human monoclonal autoantibodies.

Furthermore, the role of autoantibodies in autoimmune disease may be investigated, and the mechanism of autoimmunity may be studied in detail, e.g. by transferring autoreactive T-cells and/or target tissue or organs (e.g. human islets) to the reconstituted SCID mice. Such mice may then be used to screen for substances which have a therapeutic effect on the autoimmune disease in question.

### EXAMPLE 3

### Production of human immunoglobulin in SCD mice

### SCID mice and immunization.

C.B.-17 scid/scid (SCID) mice (male, 5-6 weeks old), with no endogenous immunoglobulin production ( < 10 ng/ml mouse IgG in serum), were injected intraperitoneally with 20 x 10⁶ Ficoll isolated PBMC per mouse. The PBMC were obtained from type 1 diabetic patients, one islet cell antibody (ICA) and 64kDa antibody negative and one ICA and 64kDa positive (Table 1). SCID mice were also reconstituted with PBMC from two healthy individuals (Table 1). Sixty days after reconstitution with PBMC, mice were immunized intraperitoneally with either neonatal rat islets (1000 islets/mouse) in incomplete Freund's adjuvant (IFA), IFA alone or affinity purified GAD from rat brain (50µg/mouse). Blood samples were obtained the day before and 19 days after immunization.

### Assay for human immunoglobulin.

Human IgG and IgM concentrations in serum were determined by an antibody capture ELISA assay: Microtiter plates, coated with goat-anti-human IgG or IgM (5 µg/ml in 50 mM NA₂CO₃/NaHCO₃-buffer) (Zymed, South San Francisco, CA), were washed (3x in PBS with 0.1% Tween 20), and incubated with serial dilutions (in PBS containing 1% BSA and 0.1% Tween 20) of the test samples for 2h; bound Ig was quantified with peroxidase-conjugated goat-anti-human IgG (H+L) (Zymed) (substrate: 0.4 mg/ml 0-phenyldiamine and 0.03% hydrogen peroxide in water). The lower detection limit of the IgG assay was 10 ng/ml. There was no detectable crossreaction to mouse Ig in this assay.

### Autoantibody detection.

To detect cytoplasmic islet cell antibodies, frozen sections of human pancreas (4 µm thick, blood group 0, acetone fixed) were incubated with sera for 90 min (mouse sera were used undiluted) and washed in PBS. Bound immunoglubulins were visualized with peroxidase-conjugated Protein A (Amersham, England) and substrate (diaminobenzidine (1 mg/ml), H₂O₂ (0.023%)) or FITC conjugated goat-anti-human IgG (Zymed). For immunoprecipitation analysis, (S³⁵)methionine labled Tx-1114 detergent phase purified cytosolic fractions, were immunoprecipitated as described in Example 1. Briefly, a cytosolic fraction, prepared from neonatal rat islet was subjected to temperature induced Tx-114 phase separation. Aliquots of the detergent phase containing amphiphilic soluble proteins were incubated overnight with sera to form immunocomplexes. The immunocomplexes were isolated by adsorption to Protein A-sepharose. The immunoprecipitates were analyzed by SDS-PAGE (10%) under reducing conditions followed by fluorography. For immunblotting, neonatal rat islets were solubilized by boiling for 3 min in 90 mM TrisHCl, 17% sucrose, 3% NaDodSO₄, and 10 mM dithiothreitol. The approximate of 1 x 10⁴ islets/10 cm of gel were subjected to 10% SDS-PAGE, electroblotted onto nitrocellulose filters and immunostained as described in J.P.Palmer, Science 222, 1983, pp 1337-1339. Bound Ig was visualized with goat-anti-human F(ab₂) IgG, conjugated to alkaline-phosphatase (Zymed) and a chromogenic substrate.

All reconstituted SCID mice produced human immunoglobulins, but in highly variable concentrations (serum huIgG 1-2816 µg/ml, huIgM 0-344 µg/ml), reaching peak levels 8-11 weeks after injection of PBMC. to detect β-cell reactive autoantibodies, sera were tested by immunohistrochemistry, immunoprecipitation, and immunoblotting analysis. Islet cell antibodies could not be detected in any of the mice reconstituted with PBMC from the 2 healthy controls, either after immunization with islet cells (n=9) in IFA or IFA alone (n=8).

None of the mice reconstituted with PBMC from the ICA negative patient were found to be ICA or 64kDa positive regardless of immunization with rat islets in IFA (n=2) or IFA alone (n=3). Mice reconstituted with PBMC from the ICA positive patient, and injected with IFA alone (n=7) were also ICA negative regardless of the huIgG concentration. In contrast, serum samples from 2 out of 10 SCID mice reconstituted with PBMC from this patient, obtained 19 days after immunization with rat islets, contained ICA of human origin staining islets with an intensity comparable to that induced by the donor patient serum at a dilution of 1:64. Surprisingly, sera from 2 SCID mice obtained 80 days after reconstitution with PBMC from the patient, were found positive for anti-nuclear-antibodies (ANA) (one had been immunized with islets and one with IFA alone). Serum from the patient was not ANA positive at any time.

Sera from the ICA positive SCID mice and an ICA negative mouse, obtained 19 days after immunization with islets immunoprecipitated the 64kDa islet cell antigen. In contrast, sera obtained from the same mice before immunization did not precipitate the 64kDa antigen. The sera precipitated the antigen with approximately the same intensity as serum from the donor patient, in spite of a much higher IgG concentration in the patient serum (8064 µg/ml in the patient compared to 512 µg/ml, 355 µg/ml and 518 µg/ml in the three SCID mice, respectively. Sera from two SCID mice, also reconstituted with PBMC from the ICA positive patient, precipitated the 64kDa antigen before immunization with rat islets, although, with significantly less intensity than the other 64kDa positive SCID mice. Immunization of one of these mice with rat islets did not influence the intensity of this binding. None of the other reconstituted SCID mice, control SCID mice not reconstituted or mice from other strains (male, age matched) precipitated the 64kDa islet cell antigen.

In immunoblotting analysis of total lysates from rat islets, a 52 kD molecule was recognized by the islet cell antibody positive, but not the islet cell antibody negative patient serum. Serum from one of the ICA and 64kDa positive SCID mice, obtained after immunization with rat islets, also recognized the 52 kD antigen in immunoblotting, whereas serum obtained from the same mouse the day before immunization did not bind the antigen. In the postimmune serum from this SCID mouse, pre-incubation with rat islets, but not human lymphocytes, reduced the binding to the 52 kD antigen, indicating that it is not a common cellular determinant.

## Claims

1. A method of producing autoantibodies which are reactive with an antigen characteristic of an autoimmune disease, the method comprising
(a) administering lymphocytes from a donor individual suffering from an autoimmune disease to immunodeficient non-human mammals,
(b) stimulating, after a period of time sufficient to permit reconstitution of immune cells in at least one of said mammals, autoreactive cells of said reconstituted immunodeficient mammal by subsequent immunization with one or more relevant target antigens,
(c) selecting a reconstituted mammal producing the desired autoantibodies,
(d) immortalizing B-lymphocytes of the selected mammal before or after isolating lymphocytes therefrom, and
(e) culturing the immortalized B-lymphocytes under conditions permitting their production of the desired autoantibodies and recovering the resulting autoantibodies from the culture.

2. A method according to claim 1 for the production of human autoantibodies, wherein the donor individual is a human donor.

3. A method according to claim 2, wherein the lymphocytes are obtained from human peripheral blood.

4. A method according to claim 1, wherein the autoimmune disease is selected from the group consisting of insulin-dependent diabetes mellitus, systemic lupus erythematosus, multiple sclerosis, thyroiditis, myasthenia gravis and rheumatoid arthritis.

5. A method according to claim 4, wherein the autoimmune disease is insulin-dependent diabetes mellitus.

6. A method according to claim 1, wherein the immunodeficient mammal is a mouse.

7. A method according to claim 6, wherein the mouse is a severe combined immunodeficient (SCID) mouse.

8. A method according to claim 1, wherein the lymphocytes administered to the mammal are derived from an IDDM patient, and wherein the reconstituted mammal is stimulated by immunization with Langerhans' islets, islet β-cells or antigens derived therefrom.

9. A method according to claim 1, wherein the desired autoantibodies are produced in a comparable or higher concentration in the reconstituted mammal than in the serum of the lymphocyte donor individual.

10. A method according to claim 9, wherein the concentration of desired autoantibodies produced by the reconstituted mammal is a least 2 times higher, more preferably at least 5 times higher, most preferably at least 10 times higher, than is found in the lymphocyte donor serum.

11. A method according to claim 10, wherein the concentration of desired autoantibodies is about 20 times higher than is found in the lymphocyte donor serum.

12. A method according to claim 1, wherein the B-lymphocytes are immortalized by inoculating the reconstituted mammal with Epstein-Barr virus before isolating B-lymphocytes from the mammal.

13. A method according to claim 1, wherein the B-lymphocytes are immortalized by Epstein-Barr virus transformation of B-lymphocytes isolated from the selected reconstituted mammal.

14. A method according to claim 1, wherein the B-lymphocytes are immortalized by fusion with a suitable immortal cell line after isolation from the reconstituted mammal.

15. A method of screening for substances with a therapeutic effect on an autoimmune disease, the method comprising administering a substance suspected of having a therapeutic effect on a specific autoimmune disease to an immunodeficient non-human mammal which has been reconstituted with immortalized B-lymphocytes produced by the method of claim 1, steps (a)-(d), and to which T-cells recognising a target cell, tissue or organ have been transferred, and determining any effect of the administered substance on the reactivity of said T-cells with said target cell, tissue or organ.

16. A method according to claim 15, wherein the lymphocytes are human lymphocytes.

17. A method according to claim 15, wherein the lymphocytes are derived from a diabetic patient.

18. A method according to claim 15, wherein the T-cells transferred to the mammal are human T-cells.

19. A method according to any of claims 15-18, wherein a target cell, tissue or organ has additionally been transferred to the mammal.

20. A method according to claim 19, wherein the target cell, tissue or organ is derived from a human donor.

21. A method according to claim 15, wherein cells or tissue from an organ affected by the autoimmune disease are additionally transplanted into the reconstituted mammal.

22. A method according to claim 21, wherein the cells or tissue are human cells or tissue.

23. A method according to claim 21 or 22, wherein the cells or tissue are islet cells or pancreatic tissue comprising islets of Langerhans.

## Patentansprüche

1. Ein Verfahren zur Herstellung von Autoantikörpern, die mit einem für eine Autoimmunerkrankung charakteristischen Antigen reaktiv sind, wobei das verfahren umfaßt, daß
(a) nicht-menschlichen immundefizienten Säugetieren Lymphocyten von einem Spender-Individuum, das an einer Autoimmunerkrankung leidet, verabreicht werden,
(b) nach einem Zeitraum, der ausreichend ist, um die Rekonstitution von Immunzellen in wenigstens einem von besagten Säugetieren zu erlauben, autoreaktive Zellen von besagtem rekonstituierten immundefizienten Säugetier durch anschließende Immunisierung mit einem oder mehreren relevanten Zielantigenen stimuliert werden,
(c) ein rekonstituiertes Säugetier ausgewählt wird, das die gewünschten Autoantikörper produziert,
(d) B-Lymphocyten des ausgewählten Säugetieres vor oder nach der Isolierung von Lymphocyten daraus immortalisiert werden und
(e) die immortalisierten B-Lymphocyten unter Bedingungen kultiviert werden, die ihre Produktion der gewünschten Autoantikörper erlauben, und die resultierenden Autoantikörper aus der Kultur gewonnen werden.

2. Ein Verfahren nach Anspruch 1, zur Herstellung von menschlichen Autoantikörpern, wobei das Spender-Individuum ein menschlicher Spender ist.

3. Ein Verfahren nach Anspruch 2, wobei die Lymphocyten aus menschlichem peripheren Blut gewonnen werden.

4. Ein Verfahren nach Anspruch 1, wobei die Autoimmunerkrankung ausgewählt ist aus der Gruppe, die aus insulinabhängigem Diabetes mellitus, systemischem Lupus erythematodes, Multipler Sklerose, Schilddrüsenentzündung, Myasthenia gravis und rheumatoider Arthritis besteht.

5. Ein Verfahren nach Anspruch 4, wobei die Autoimmunerkrankung insulinabhängiger Diabetes mellitus ist.

6. Ein Verfahren nach Anspruch 1, wobei das immundefiziente Säugetier eine Maus ist.

7. Ein Verfahren nach Anspruch 6, wobei die Maus eine Maus mit schwerem kombinierten Immundefekt (SCID) ist.

8. Ein Verfahren nach Anspruch 1, wobei die dem Säugetier verabreichten Lymphocyten aus einem IDDM-Patienten gewonnen werden und wobei das rekonstituierte Säugetier durch Immunisierung mit Langerhans'schen Inseln, Insel-β-Zellen oder daraus gewonnenen Antigenen stimuliert wird.

9. Ein Verfahren nach Anspruch 1, wobei die gewünschten Autoantikörper in einer vergleichbaren oder höheren Konzentration im rekonstituierten Säugetier als im Serum des Lymphocyten-Spender-Individuums produziert werden.

10. Ein Verfahren nach Anspruch 9, wobei die Konzentration an von dem rekonstituierten Säugetier produzierten gewünschten Autoantikörpern wenigstens 2-mal höher, bevorzugter wenigstens 5-mal höher, am bevorzugtesten wenigstens 10-mal höher ist, als sie im Lymphocyten-Spenderserum anzutreffen ist.

11. Ein Verfahren nach Anspruch 10, wobei die Konzentration an gewünschten Autoantikörpern etwa 20-mal höher ist, als sie im Lymphocyten-Spenderserum anzutreffen ist.

12. Ein Verfahren nach Anspruch 1, wobei die B-Lymphocyten immortalisiert werden, indem das rekonstituierte Säugetier vor der Isolierung von B-Lymphocyten aus dem Säugetier mit Epstein-Barr-Virus inokuliert wird.

13. Ein Verfahren nach Anspruch 1, wobei die B-Lymphocyten durch Epstein-Barr-Virus-Transformation von B-Lymphocyten immortalisiert werden, die aus dem ausgewählten rekonstituierten Säugetier isoliert worden sind.

14. Ein Verfahren nach Anspruch 1, wobei die B-Lymphocyten durch Fusion mit einer geeigneten immortalen Zellinie nach Isolierung aus dem rekonstituierten Säugetier immortalisiert werden.

15. Ein Verfahren zum Screening auf Substanzen mit einer therapeutischen Wirkung auf eine Autoimmunerkrankung, wobei das Verfahren umfaßt, daß eine Substanz, von der man vermutet, daß sie eine therapeutische Wirkung auf eine spezifische Autoimmunerkrankung besitzt, einem nicht-menschlichen immundefizienten Säugetier verabreicht wird, das mit immortalisierten B-Lymphocyten rekonstituiert worden ist, die mit dem Verfahren nach Anspruch 1, Schritte (a)-(d), produziert worden sind, und auf das T-Zellen, die eine Zielzelle, ein Zielgewebe oder ein Zielorgan erkennen, übertragen worden sind, und das jegliche Wirkung der verabreichten Substanz auf die Reaktivität besagter T-Zellen mit besagter Zielzelle, besagtem Zielgewebe oder besagtem Zielorgan bestimmt wird.

16. Ein Verfahren nach Anspruch 15, wobei die Lymphocyten menschliche Lymphocyten sind.

17. Ein Verfahren nach Anspruch 15, wobei die Lymphocyten aus einem Diabetes-Patienten gewonnen sind.

18. Ein Verfahren nach Anspruch 15, wobei die T-Zellen, die auf das Säugetier übertragen worden sind, menschliche T-Zellen sind.

19. Ein Verfahren nach einem der Ansprüche 15-18, wobei eine Zielzelle, ein Zielgewebe oder ein Zielorgan zusätzlich auf das Säugetier übertragen worden ist.

20. Ein Verfahren nach Anspruch 19, wobei die Zielzelle, das Zielgewebe oder das Zielorgan von einem menschlichen Spender gewonnen ist.

21. Ein Verfahren nach Anspruch 15, wobei Zellen oder Gewebe aus einem Organ, die (das) von der Autoimmunerkrankung befallen sind (ist), zusätzlich in das rekonstituierte Säugetier transplantiert werden (wird).

22. Ein Verfahren nach Anspruch 21, wobei die Zellen oder das Gewebe menschliche Zellen oder menschliches Gewebe sind (ist).

23. Ein Verfahren nach Anspruch 21 oder 22, wobei die Zellen oder das Gewebe Insel-Zellen oder Bauchspeicheldrüsengewebe, das Langerhans'sche Inseln umfaßt, sind (ist).

## Revendications

1. Procédé pour la production d'auto-anticorps qui réagissent à l'antigène caractéristique d'une maladie auto-immune, le procédé comprenant les étapes consistant à
(a) administrer des lymphocytes à partir d'un donneur individuel souffrant d'une maladie auto-immune à des mammifères non humains immunodéficients,
(b) stimuler, après une période de temps suffisante pour permettre la reconstitution de cellules immunitaires chez au moins l'un de ces mammifères, les cellules auto-réactives de ce mammifère immunodéficient reconstitué par immunisation subséquente avec un ou plusieurs antigènes cibles appropriés,
(c) sélectionner un mammifère reconstitué produisant les auto-anticorps souhaités,
(d) immortaliser les lymphocytes B du mammifère sélectionné avant ou après l'isolation des lymphocytes de celui-ci, et
(e) mettre en culture les lymphocytes B immortalisés dans des conditions permettant leur production d'auto-anticorps souhaités et récupérer les auto-anticorps résultants de la culture.

2. Procédé selon la revendication 1 pour la production d'auto-anticorps humains dans lequel le donneur individuel est un donneur humain.

3. Procédé selon la revendication 2, dans lequel les lymphocytes sont obtenus à partir de sang périphérique humain.

4. Procédé selon la revendication 1, dans lequel la maladie auto-immune est choisie dans le groupe constitué par le diabète sucré insulino-dépendant, le lupus erythematosus systémique, la sclérose en plaques, la thyroïdite, la myasthénie profonde et l'arthrite rhumatoïde.

5. Procédé selon la revendication 4, dans lequel la maladie auto-immune est le diabète sucré insulino-dépendant.

6. Procédé selon la revendication 1, dans lequel le mammifère immunodéficient est une souris.

7. Procédé selon la revendication 6, dans lequel la souris est une souris atteinte d'immunodéficience combinée grave (SCID).

8. Procédé selon la revendication 1, dans lequel les lymphocytes administrés au mammifère sont dérivés d'un patient IDDM et dans lequel le mammifère reconstitué est stimulé par immunisation ave des îlots de Langerhans, des cellules β d'îlots ou des antigènes dérivés de ceux-ci.

9. Procédé selon la revendication 1, dans lequel les auto-anticorps souhaités sont produits en une concentration comparable ou supérieure dans le mammifère reconstitué à celle du sérum du donneur individuel de lymphocytes.

10. Procédé selon la revendication 9, dans lequel la concentration d'auto-anticorps souhaités produits par le mammifère reconstitué est au moins 2 fois supérieure, de façon plus préférée d'au moins 5 fois supérieure, de façon la plus préférée d'au moins 10 fois supérieure qu'elle n'est trouvée dans les lymphocytes du sérum du donneur.

11. Procédé selon la revendication 10, dans lequel la concentration des auto-anticorps souhaités est d'environ 20 fois supérieure à celle trouvée dans le sérum lymphocyte du donneur.

12. Procédé selon la revendication 1, dans lequel les lymphocytes B sont immortalisés par inoculation du mammifère reconstitué avec le virus Epstein-Barr avant d'isoler les lymphocytes B du mammifère.

13. Procédé selon la revendication 1, dans lequel les lymphocytes B sont immortalisés par la transformation par le virus d'Epstein-Barr des lymphocytes B isolés du mammifère reconstitué sélectionné.

14. Procédé selon la revendication 1, dans lequel les lymphocytes B sont immortalisés par fusion avec une lignée de cellules immortelles appropriées après isolation du mammifère reconstitué.

15. Procédé de criblage de substances ayant un effet thérapeutique sur une maladie auto-immune, le procédé comprenant l'administration d'une substance que l'on soupçonne avoir un effet thérapeutique sur une maladie auto-immune spécifique à un mammifère non humain immunodéficient qui a été reconstitué par lymphocytes B immortalisés produits par le procédé de la revendication 1, les étapes (a)-(d)) et auquel on a transféré les cellules T reconnaissant une cellule cible, tissu ou organe et déterminant tout effet de la substance administrée sur la réactivité des cellules T avec l'organe, le tissu ou la cellule cible.

16. Procédé selon la revendication 15, dans lequel les lymphocytes sont des lymphocytes humains.

17. Procédé selon la revendication 15, dans lequel les lymphocytes sont dérivés d'un patient diabétique.

18. Procédé selon la revendication 15, dans lequel les cellules T transférées au mammifère sont des cellules T humaines.

19. Procédé selon l'une quelconque des revendications 15-18, dans lequel une cellule cible, un tissu ou un organe ont de plus été transférés au mammifère.

20. Procédé selon la revendication 19, dans lequel la cellule cible, tissu ou organe est dérivé d'un donneur humain.

21. Procédé selon la revendication 15, dans lequel les cellules ou tissus provenant d'un organe affecté par la maladie auto-immune sont de plus transplantées dans le mammifère reconstitué.

22. Procédé selon la revendication 21, dans lequel les cellules ou tissus sont des cellules ou tissus humains.

23. Procédé selon la revendication 21 ou 22, dans lequel les cellules ou tissus sont des cellules d'îlots ou de tissu pancréatique comprenant les îlots de Langerhans.
